**Europäisches Patentamt**

(19) **European Patent Office**

**Office européen des brevets**

(11) Veröffentlichungsnummer: **0 182 017**

**B1**

---

(12) **EUROPÄISCHE PATENTSCHRIFT**

(45) Veröffentlichungstag der Patentschrift: 29.03.89

(51) Int. Cl.⁴: **C 07 C 143/10**

(21) Anmeldenummer: **85110940.5**

(22) Anmeldetag: **30.08.85**

(54) **Oberflächenaktive Kondensationsprodukte.**

---

(30) Priorität: **22.11.84 DE 3442579**

(43) Veröffentlichungstag der Anmeldung: **28.05.86 Patentblatt 86/22**

(45) Bekanntmachung des Hinweises auf die Patenterteilung: **29.03.89 Patentblatt 89/13**

(84) Benannte Vertragsstaaten: **BE CH DE FR GB IT LI NL SE**

(56) Entgegenhaltungen: **GB-A-853 590**

(73) Patentinhaber: **Akzo Patente GmbH, Postfach 10 01 49 Kasinostrasse 19- 23, D-5600 Wuppertal- 1 (DE)**

(72) Erfinder: **Zok, Claus- Peter, Chem.- Ing., Nelly- Pütz- strasse 29, D-5162 Niederzier 2 (DE)**

(74) Vertreter: **Pfeiffer, Ernst, Akzo NV Akzo Patents Department P.O. Box 9300, NL- 6800 SB Arnhem (NL)**

---

LIBER, STOCKHOLM 1989

**Beschreibung**

Die Erfindung betrifft ein Verfahren zur Herstellung von oberflächenaktiven Kondensationsprodukten durch Verestern von Carbonsäuren mit Salzen von Hydroxyalkansulfonsäuren.

Die Herstellung derartiger Kondensationsprodukte ist bereits seit langem bekannt. So wird in dem Buch von August Chwala, Textilhilfsmittel, Ihre Chemie, Kolloid-Chemie und Anwendung Verlag J. Springer, Wien, 1939 auf den Seiten 173 bis 174 ein solches Verfahren beschrieben. Dabei wird Ölsäure oder Ölsäurechlorid mit hydroxyäthansulfonsaurem Natrium verestert. Der dabei entstehende Ester der Ölsäure und der Hydroxyäthansulfonsäure (Isäthionsäure), auch Igepon genannt, ist oberflächenaktiv und hat sich z. B. als Waschmittel für Proteinfasern und Kunstfasern aus regenerierter Cellulose bewährt.

Die Bedeutung derartiger Kondensationsprodukte hat im Laufe der Zeit zugenommen, und so gibt es zahlreiche Hinweise auf derartige Herstellungsverfahren, sei es in der Patentliteratur oder in Fachzeitschriften.

So wird z. B. in der US-PS-2 635 103 ein Verfahren beschrieben, bei der ein Fettsäuregemisch, in dem der Hauptbestandteil Laurinsäure ist, mit 2-hydroxyäthansulfonsaurem Natrium umgesetzt wird. Die US-PS-2 857 370 lehrt die Umsetzung einer Reihe von Carbonsäuren mit hydroxyalkansulfonsauren Salzen in Gegenwart eines borhaltigen Katalysators. In der europäischen Patentanmeldung Nr. 0 067 624 wird ein Verfahren zur Herstellung derartiger Kondensationsprodukte beschrieben, bei dem 2-hydroxyalkansulfonsaures Alkali mit einer Fettsäure verestert wird, wobei ein Veresterungskatalysator und zusätzlich ein Alkalimetallhydroxid oder ein Alkalimetallsalz einer Carbonsäure eingesetzt wird. Dadurch soll einer Verfärbung des Reaktionsproduktes vorgebeugt werden.

Es hat sich gezeigt, daß es zweckmäßig ist, bei dieser Reaktion für eine gute Homogenisierung der Reaktionsmischung zu sorgen, d.h. es muß während der Umsetzung ständig gerührt werden. Da die Reaktion im allgemeinen einige Stunden dauert und die Viskosität mit zunehmender Veresterung stark ansteigt, ist ein verhältnismäßig hoher Energieaufwand für das Homogenisieren erforderlich. Dies ist besonders gegen Ende der Reaktion ein Problem, wenn der Gehalt an Kondensationsprodukt nahezu seinen Endwert erreicht hat und überschüssige Carbonsäure weitgehend abdestilliert wird. Daher ist es notwendig, entsprechend robuste Rührer einzusetzen, die in der Lage sind, die hochviskose Reaktionsmasse noch ausreichend zu durchmischen.

Es besteht deshalb noch ein Bedürfnis nach einem verbesserten Verfahren, das in einfacher, wirtschaftlicher Weise zu Kondensationsprodukten der oben erwähnten Art führt. Aufgabe der Erfindung ist es ferner ein Verfahren zur Verfügung zu stellen, bei dem sich die Reaktionsmasse während der gesamten Reaktion und insbesondere auch gegen Ende der Reaktion leichter homogenisieren läßt und bei dem es möglich ist, mit Rührwerkzeugen einfacherer Art zu arbeiten, die nicht auf höchste Beanspruchung ausgelegt sein müssen. Aufgabe der Erfindung ist es ferner ein Verfahren zur Verfügung zu stellen, das zu Kondensationsprodukten der oben erwähnten Art führt, die gute oberflächenaktive Eigenschaften aufweisen und die sich gut zu Fertigprodukten, wie Detergentien in kompakter oder pulvriger Form verarbeiten lassen.

Diese Aufgabe wird gelöst durch ein Verfahren zur Herstellung von oberflächenaktiven Kondensationsprodukten durch Verestern von Carbonsäuren mit Salzen von Hydroxyalkansulfonsäuren, das dadurch gekennzeichnet ist, daß man Carbonsäuren der allgemeinen Formel RCOOH, wobei R ein gesättigter und/oder ungesättigter Kohlenwasserstoffrest mit 7 bis 31 Kohlenstoffatomen ist, mit einem Salz der Formel $HO(CH_2)_n SO_3X$, wobei n Werte von 2 - 4 annehmen kann und X ein Alkalimetall oder $NH_4$ bedeutet, in Gegenwart eines Konsistenzreglers verestert, wobei man als Konsistenzregler Ester synthetischer oder natürlicher Fettsäuren verwendet, vorzugsweise deren Methylester. Als Konsistenzregler sind besonders geeignet Ester der allgemeinen Formel R'COOR'', wobei bei R' ein gesättigter und/oder ungesättigter Rest mit 7 bis 17 Kohlenstoffatomen bedeutet und R'' $CH_3$ oder $C_2H_5$ bedeutet. Sehr geeignet sind Methylester von aliphatischen Carbonsäuren mit 12 bis 18 Kohlenstoffatomen. Der Konsistenzregler wird im allgemeinen in Mengen von 1 bis 50 Gew.-%, insbesondere in Mengen von 10 bis 20 % verwendet; dabei werden die Gewichtsprozente bezogen auf die Menge der Ausgangsstoffe, d.h. der Carbonsäuren, des hydroxyalkansulfonsauren Salzes, des Konsistenzreglers und gegebenenfalls weiterer Zusatzstoffe.

Zur Umsetzung werden bevorzugt Carbonsäuren mit 8 bis 18 Kohlenstoffatomen oder deren Gemische verwendet, insbesondere Kokosfettsäuren. Als Salze von Hydroxyalkansulfonsäuren werden vorzugsweise Alkalisalze der 2-Hydroxyäthansulfonsäure, insbesondere das 2-hydroxyäthansulfonsaure Natrium verwendet.

Die Reaktion kann in Gegenwart eines Katalysators durchgeführt werden, der vorzugsweise in Mengen von 0,1 bis 5 Gew.-% zugegen ist. Besonders geeignet sind 0,1 bis 2 Gew.-% Phosphorsäure.

In einer besonders vorteilhaften Ausführungsform der Erfindung wird die Reaktionsmasse nach der Veresterung ohne wesentliche Abkühlung in Wasser gelöst, anschließend wird die erhaltene Lösung sprühgetrocknet.

Die Veresterung wird vorzugsweise bei Temperaturen von etwa 220 bis 245° C im Vakuum durchgeführt, insbesondere bei 225 bis 235° C.

Bevorzugt wird die Veresterung unter Schutzgas, insbesondere unter Stickstoff durchgeführt.

Als Konsistenzregler im Rahmen der Erfindung ist zu verstehen ein Mittel, das die Viskosität des Reaktionsgemisches herabsetzt. Bevorzugt werden solche Konsistenzregler, die nach Beendigung der Umsetzung weitgehend aus dem Endprodukt entfernt werden können oder die im Endprodukt keine schädlichen oder unerwünschten Nebenwirkungen entfalten.

Die Durchführung des erfindungsgemäßen Verfahrens kann auf folgende Weise geschehen:

Ein Salz einer entsprechenden Hydroxyalkansulfonsäure, z. B. 2-hydroxyäthansulfonsaures Natrium wird mit der Carbonsäure oder einem Carbonsäuregemisch und dem Konsistenzregler vermischt, wobei gegebenenfalls ein Katalysator zugeführt wird. Weitere übliche Zusätze sind möglich. Die Reihenfolge der Zugabe der Ausgangsstoffe in das Reaktionsgefäß kann beliebig erfolgen. Das Reaktionsgefäß ist mit einem entsprechenden Rührer versehen. Sodann wird auf eine Temperatur von beispielsweise 230 bis 235° C erhitzt. Bei dieser Temperatur wird das Reaktionsgemisch einige Stunden gehalten, z. B. 4 Stunden, bis die Veresterung weitgehend abgeschlossen ist. Sowohl beim Aufheizen als auch während der Veresterung wird gerührt. Sodann wird Vakuum angelegt und der Konsistenzregler zusammen mit überschüssiger Fettsäure und dem Rest Wasser zu einem großen Teil abdestilliert. Dies ist im allgemeinen der Fall, wenn sich ein Vakuum von etwa 35 bis 40 mbar einstellt. Während der Destillation wird das Gemisch auf einer Temperatur von etwa 230° C gehalten. Anschließend wird das noch heiße Gemisch in vorgelegtes Wasser gegossen, so daß beispielsweise eine etwa 30 bis 40 %-ige Lösung entsteht. Diese Lösung kann sofort sprühgetrocknet werden.

Selbstverständlich kann die Schmelze auch ohne Lösen in Wasser und ohne Einschalten einer Sprühtrocknung weiterverarbeitet werden, z. B. durch Gießen, Mahlen oder Granulieren.

Die Umsetzung des sulfonsauren Salzes mit den Carbonsäuren kann in stöchiometrischen Verhältnis erfolgen, d.h. daß auf 1 Mol Salz 1 Mol Säure eingesetzt wird. Vorzugsweise wird jedoch mit einem Überschuß an Säure gearbeitet, z. B. mit einem Molverhältnis hydroxyalkansulfonsaurem Salz zu Fettsäure von 1 : 1,2 bis 1 : 1,4.

Die Reaktionszeit hängt selbstverständlich von den Ausgangsstoffen und auch von der gewählten Temperatur ab. Im allgemeinen genügen bei Temperaturen um 230° C z. B. Zeiten von 2 bis 5 Stunden, insbesondere von 3,5 bis 4 Stunden, um eine ausreichende Veresterung zu erhalten. Die Reaktion wird vorzugsweise unter Normaldruck durchgeführt, wobei es sich empfiehlt, unter einer Schutzgasatmosphäre, insbesondere unter Stickstoffatmosphäre zu arbeiten.

Die Veresterung kann mit oder ohne Katalysator durchgeführt werden. Bei Einsatz von Katalysatoren reichen im allgemeinen Mengen von 0,1 bis 5 Gew.-% aus. Sehr geeignet als Katalysatoren sind Säuren des Phosphors, insbesondere die Orthophosphorsäure und die hypophosphorige Säure. Die Säuren des Phosphors können auch im Gemisch verwendet werden.

Nach Beendigung der Veresterung kann das Produkt in Formen gegossen werden, in denen es abkühlt. Es ist auch möglich, die noch heiße Schmelze in Wasser zu gießen, wobei eine Lösung des oberflächenaktiven Mittels entsteht. Das Wasser dieser Lösung kann durch Sprühtrocknen entfernt werden.

Je nach den geforderten Reinheitsansprüchen kann das Produkt direkt ohne weitere Aufbereitung verarbeitet werden oder man kann auch weitere Reinigungsprozesse wie Umkristallisieren oder das eben erwähnte Sprühtrocknen durchführen.

Als Carbonsäuren können übliche synthetische oder natürlich vorkommende Carbonsäuren verwendet werden; sehr geeignet sind auch Gemische von Carbonsäuren mit unterschiedlichen Kettenlängen, wie sie aus natürlichen Produkten wie Kokosöl gewonnen werden können. In einer besonders vorteilhaften Ausführungsform werden deshalb Kokosfettsäuren eingesetzt.

Es war besonders überraschend, daß sich mit dem erfindungsgemäßen Verfahren auf einfache Weise die gewünschten oberflächenaktiven Kondensationsprodukte herstellen lassen. Durch die Mitverwendung des Konsistenzreglers wird die Viskosität während der Veresterung bis zum Abschluß der Umsetzung erheblich niedriger gehalten, als das bei einer Veresterung ohne die Mitverwendung der Konsistenzregler der Fall wäre. Dadurch ist erheblich weniger Rührenergie erforderlich; die Durchmischung und Homogenisierung des Reaktionsgemisches erfolgt leichter und vollständiger, was sich vorteilhaft auf die Umsetzung und die Gleichmäßigkeit des erhaltenen Produktes auswirkt. Besondere Hochleistungsrührer oder Kneter brauchen nicht eingesetzt werden. So lassen sich einfache Balkenrührer einsetzen.

Der durch Abdestillieren entfernte Konsistenzregler kann für weitere Reaktionen wieder eingesetzt werden. Das gleiche gilt für die abdestillierten Fettsäuren.

Beim Sprühtrocknen einer wäßrigen Lösung wird weiterer Konsistenzregler und freie Carbonsäure, die im Rohprodukt noch vorhanden sind, entfernt, so daß ein qualitativ gutes Produkt erhalten wird, das sich vorteilhaft als Rohstoff für die Waschmittelherstellung, als Detergens und als Rohstoff für kosmetische Formulierungen, insbesondere Seifen eignet.

Besonders überraschend ist, daß der erfindungsgemäße Einsatz des viskositätserniedrigenden Konsistenzreglers es ermöglicht, das Endprodukt problemlos direkt in Wasser zu lösen und auf diese Weise die vorteilhafte Sprühtrocknung des Produkts durchführen zu können. Man erhält auf diese Weise ein rieselfähiges Produkt mit hervorragenden Eigenschaften, das sehr vielseitig einsetzbar ist.

**Patentansprüche**

1. Verfahren zur Herstellung von oberflächenaktiven Kondensationsprodukten durch Verestern von Carbonsäuren mit Salzen von Hydroxyalkansulfonsäuren, dadurch gekennzeichnet, daß man Carbonsäuren der allgemeinen Formel RCOOH, wobei R ein gesättigter und/oder ungesättigter Kohlenwasserstoffrest mit 7 bis 31 Kohlenstoffatomen ist, mit einem Salz der Formel $HO-(CH_2)_n-SO_3X$, wobei n Werte von 2 - 4 annehmen kann und X ein Alkalimetall oder $NH_4$ bedeutet, in Gegenwart eines Konsistenzreglers verestert, wobei man als Konsistenzregler Ester synthetischer oder natürlicher Fettsäuren verwendet.

2. Verfahren nach Anspruch 1, dadurch gekennzeichnet, daß man Methylester verwendet.

3. Verfahren nach Anspruch 1, dadurch gekennzeichnet, daß man Ester der allgemeinen Formel R'COOR'' verwendet, wobei R' ein gesättigter und/oder ungesättigter Rest mit 7 bis 17 Kohlenstoffatomen und R'' $CH_3$ oder $C_2H_5$ bedeutet.

4. Verfahren nach Anspruch 3, dadurch gekennzeichnet, daß man Methylester von aliphatischen Carbonsäuren mit 12 bis 18 Kohlenstoffatomen verwendet.

5. Verfahren nach einem der Ansprüche 1 bis 4, dadurch gekennzeichnet, daß man 1 bis 50 Gew.-%, bezogen auf die Menge der Ausgangsstoffe des Konsistenzreglers verwendet.

6. Verfahren nach einem der Ansprüche 1 bis 5, dadurch gekennzeichnet, daß man Gemische der Konsistenzregler verwendet.

7. Verfahren nach einem der Ansprüche 1 bis 6, dadurch gekennzeichnet, daß man zur Veresterung aliphatische Carbonsäuren mit 8 bis 18 Kohlenstoffatomen verwendet.

8. Verfahren nach einem der Ansprüche 1 bis 7, dadurch gekennzeichnet, daß man zur Veresterung Kokosfettsäuren verwendet.

9. Verfahren nach einem der Ansprüche 1 bis 8, dadurch gekennzeichnet, daß man zur Veresterung Gemische der Carbonsäuren verwendet.

10. Verfahren nach einem der Ansprüche 1 bis 9, dadurch gekennzeichnet, daß man als Salz von Hydroxyalkansulfonsäuren 2-hydroxyäthansulfonsaures Natrium verwendet.

11. Verfahren nach einem der Ansprüche 1 bis 10, dadurch gekennzeichnet, daß man die Veresterung in Gegenwart von 0,1 bis 5 Gew.-% eines Veresterungskatalysators durchführt.

12. Verfahren nach Anspruch 11, dadurch gekennzeichnet, daß man als Katalysator 0,1 bis 2 Gew.-% Phosphorsäure verwendet.

13. Verfahren nach einem der Ansprüche 1 bis 12, dadurch gekennzeichnet, daß man die Reaktionsmasse nach der Veresterung ohne wesentliche Abkühlung in Wasser löst und die erhaltene Lösung anschließend sprühtrocknet.

14. Verfahren nach einem der Ansprüche 1 bis 13, dadurch gekennzeichnet, daß man die Veresterung bei einer Temperatur von 220 bis 245° C im Vakuum durchführt.

15. Verfahren nach Anspruch 14, dadurch gekennzeichnet, daß man die Veresterung bei einer Temperatur von 225 bis 235° C durchführt.

**Claims**

1. A process for the production of surface-active condensates by esterification of carboxylic acids with salts of hydroxyalkanesulfonic acids, characterized in that carboxylic acids corresponding to the general formula RCOOH, where R is a saturated and/or unsaturated hydrocarbon radical containing from 7 to 31 carbon atoms, are esterified with a salt corresponding to the formula $HO-(CH_2)_n-SO_3X$, where n may assume a value of from 2 to 4 and X is an alkali metal or $NH_4$, in the presence of a viscosity regulator, synthetic or natural fatty acids being used as the consistency regulator.

2. A process as claimed in claim 1, characterized in that methyl esters are used.

3. A process as claimed in claim 1, characterized in that esters corresponding to the general formula R'COOR'', where R' is a saturated and/or unsaturated radical containing 7 to 17 carbon atoms and R'' represents $CH_3$ or $C_2H_5$, are used.

4. A process as claimed in claim 3, characterized in that methyl esters of aliphatic carboxylic acids containing 12 to 18 carbon atoms are used.

5. A process as claimed in any of claims 1 to 4, characterized in that the viscosity regulator is used in a quantity of 1 to 50 % by weight, based on the quantity of starting materials.

6. A process as claimed in any of claims 1 to 5, characterized in that mixtures of the viscosity regulators are used.

7. A process as claimed in any of claims 1 to 6, characterized in that aliphatic carboxylic acids containing 8 to 18 carbon atoms are used for the esterification.

8. A process as claimed in any of claims 1 to 7, characterized in that coconut oil fatty acids are used for the esterification.

9. A process as claimed in any of claims 1 to 8, characterized in that mixtures of the carboxylic acids are used for the esterification.

10. A process as claimed in any of claims 1 to 9, characterized in that sodium 2-hydroxyethanesulfonate is used as the salt of hydroxyalkanesulfonic acids.

11. A process as claimed in any of claims 1 to 10, characterized in that the esterification is carried out in the presence of 0.1 to 5 % by weight of an esterification catalyst.

12. A process as claimed in claim 11, characterized in that 0.1 to 2 % by weight phosphoric acid is used as the catalyst.

13. A process as claimed in any of claims 1 to 12, characterized in that, after esterification, the reaction mixture is dissolved in water without significant cooling and the solution obtained is subsequently spray-dried.

14. A process as claimed in any of claims 1 to 13, characterized in that the esterification is carried out in vacuo at a temperature of 220 to 245°C.

15. A process as claimed in claim 14, characterized in that the esterification is carried out at a temperature of 225 to 235°C.

**Revendications**

1. Procédé de préparation de produits de condensation tensio-actifs, par estérification d'acides carboxyliques avec des sels d'acides hydroxyalcane sulfoniques, caractérisé en ce que l'on estérifie des acides carboxyliques de formule générale RCOOH, dans laquelle R représente un radical hydrocarboné saturé et/ou insaturé comportant 7 à 31 atomes de carbone, avec un sel de formule HO-$(CH_2)_n$-$SO_3$X, dans laquelle n peut prendre les valeurs de 2 à 4 et X représente un métal alcalin ou $NH_4$, en présence d'un agent de régulation de consistance, l'agent de régulation de consistance utilisé étant un ester d'acides gras synthétiques ou naturels.

2. Procédé conforme à la revendication 1, caractérisé en ce que l'on utilise un ester méthylique.

3. Procédé conforme à la revendication 1, caractérisé en ce que l'on utilise un ester de formule générale R'COOR'', dans laquelle R' représente un radical saturé et/ou insaturé comportant 7 à 17 atomes de carbone, et R'' représente $CH_3$ ou $C_2H_5$.

4. Procédé conforme à la revendication 3, caractérisé en ce que l'on utilise un ester méthylique d'acides carboxyliques aliphatiques comportant 12 à 18 atomes de carbone.

5. Procédé conforme à l'une des revendications 1 à 4, caractérisé en ce que l'on utilise de 1 à 50 % en poids de l'agent de régulation de consistance, par rapport à la quantité des produits de départ.

6. Procédé conforme à l'une des revendications 1 à 5, caractérisé en ce que l'on utilise des mélanges d'agents de régulation de consistance.

7. Procédé conforme à l'une des revendications 1 à 6, caractérisé en ce que l'on utilise pour l'estérification des acides carboxyliques aliphatiques comportant 8 à 18 atomes de carbone.

8. Procédé conforme à l'une des revendications 1 à 7, caractérisé en ce que l'on utilise pour l'estérification les acides gras de coco.

9. Procédé conforme à l'une des revendications 1 à 8, caractérisé en ce que l'on utilise pour l'estérification des mélanges d'acides carboxyliques.

10. Procédé conforme à l'une des revendications 1 à 9, caractérisé en ce que l'on utilise, comme sel d'acides hydroxyalcanesulfoniques, le 2-hydroxyéthanesulfonate de sodium.

11. Procédé conforme à l'une des revendications 1 à 10, caractérisé en ce que l'on réalise l'estérification en présence de 0,1 à 5 % en poids d'un catalyseur d'estérification.

12. Procédé conforme à la revendication 11, caractérisé en ce que l'on utilise comme catalyseur 0,1 à 2 % en poids d'acide phosphoré.

13. Procédé conforme à l'une des revendications 1 à 12, caractérisé en ce que l'on dissout la masse réactionnelle, après l'estérification, pratiquement sans refroidissement, dans de l'eau et que la solution obtenue est ensuite séchée par pulvérisation.

14. Procédé conforme à l'une des revendications 1 à 13, caractérisé en ce que l'on effectue l'estérification sous vide, à une température de 220 à 245°C.

15. Procédé conforme à la revendication 14, caractérisé en ce que l'on effectue l'estérification à une température de 225 à 235°C.